# EUROPEAN PATENT SPECIFICATION

(11) **EP 3 673 270 B1**
(45) Date of publication and mention of the grant of the patent: **11.12.2024**
(21) Application number: 18756245.9
(22) Date of filing: 21.08.2018
(51) Int. Cl.: G01N 33/68

(54) **DIAGNOSTIC METHOD OF A SKIN SHOWING SIGNS OF AGING**
DIAGNOSTISCHES VERFAHREN FÜR EINE HAUT MIT ANZEICHEN VON ALTERUNG
PROCÉDÉ DE DIAGNOSTIC D'UNE PEAU PRÉSENTANT DES SIGNES DE VIEILLISSEMENT

(30) Priority: 23.08.2017 FR 1757817
(43) Date of publication of application: 01.07.2020
(73) Proprietor: L'OREAL, 75008 Paris (FR)
(72) Inventor: KANANI, Sandra, 93601 Aulnay-Sous-Bois (FR); PIFFAUT, Virginie, 93601 Aulnay-Sous-Bois (FR); FOUCHER, Aude, 93601 Aulnay-Sous-Bois (FR); DONOVAN, Mark, 93601 Aulnay-Sous-Bois (FR)
(74) Representative: Lavoix
(86) International application number: PCT/EP2018/072569
(87) International publication number: WO 2019/038290

(56) References cited:
- WO-A1-2012/038929
- FR-A1- 2 968 560
- HARDMAN MATTHEW J ET AL: "Estrogen, not intrinsic aging, is the major regulator of delayed human wound healing in the elderly", GENOME BIOLOGY, BIOMED CENTRAL LTD., LONDON, GB, vol. 9, no. 5, 13 May 2008 (2008-05-13), pages R80, XP021041623, ISSN: 1465-6906
- CHESNEAU V ET AL: "Functional Human Insulin-Degrading Enzyme Can Be Expressed in Bacteria", PROTEIN EXPRESSION AND PURIFICAT, ACADEMIC PRESS, SAN DIEGO, CA, vol. 19, no. 1, 1 June 2000 (2000-06-01), pages 91 - 98, XP004435520, ISSN: 1046-5928, DOI: 10.1006/PREP.2000.1217
- WILLIAM C. DUCKWORTH ET AL: "Insulin-Degrading Activity in Wound Fluid", JOURNAL OF CLINICAL ENDOCRINOLOGY AND METABOLISM, vol. 89, no. 2, 1 February 2004 (2004-02-01), US, pages 847 - 851, XP055476147, ISSN: 0021-972X, DOI: 10.1210/jc.2003-031371

## Description

This invention relates to a diagnostic method of a skin showing signs of aging.

During aging the skin is on the overall subjected to much damage, reflections of functional and structural alterations and of degradations of all of its compartments (epidermis, dermis-epidermis junction, dermis, dermis-hypodermis junction), at the same time as a drop in the quality of the vascularisation. At the cellular level, many metabolic functions are affected. The epidermis becomes thinner with age, the keratinocytes lose their renewal and repair capacity, the synthesis of hyaluronic acid, of proteoglycans collapses with age, the quality of the cohesion of the dermis-epidermis junction drops making skin tissue thinner and more fragile, the thickness of the epidermis is thinner, the dermis-epidermis junction loses its invaginations major characteristics of a young skin. The same applies at the dermic level at which fibroblasts synthesize fewer proteins or fundamental matrix molecules in its organization (collagens, particularly proteoglycanes). The hypodermis is also affected. All of these events weaken all of the skin which becomes less firm, less elastic, fragile and has difficulty in retaining a correct level of hydration (disturbance of the barrier function). The skin can also lose its shine, have alterations in its microrelief causing the appearance of a weathered texture, an appearance of wrinkles and lines, have an increase in the heterogeneity of the diameter of the pores, all signs that are perceived as "poor health" or fatigue and that modify the perception of the apparent age. Furthermore, the alteration of all of the properties of the skin can be accentuated by different secondary factors, such as the consequence of different physiological disorders, environmental aggressions, such as pollution or radiation such as ultraviolet rays, extrinsic factors, such as tobacco, variations in organization, in the density of the hypodermis, consecutive to a modification in the body mass, pregnancy, obesity, cellulite, or indeed disorganization of the components of the extracellular matrix resulting for example from stretch marks, scars.

From the above, the importance of finding biomarkers that make it possible to detect the signs of aging of the skin is understood and in particular when the latter are not yet visible, so as to be able to reduce and/or slow down the progression of these signs of aging of the skin. More particularly, as the aging of the skin can be materialized by one or several of the various signs mentioned hereinabove, there is a substantial need for specific biomarkers of one or more of these signs, so as to suitably target them in a given subject. Dermicidin has already been described a biomarker for ageing skin and/or signs of skin ageing which are possibly associated with skin dryness (WO2012/038929 A1).The aim of the invention is to meet this need by providing a new biomarker.

The applicant surprisingly discovered that the expression of the gene encoding the insulin-degrading enzyme (IDE), on the skin, is correlated with the presence of signs of aging of the skin, more particularly with elastosis, the appearance of crow's feet wrinkles and the appearance of wrinkles under the eyes.

The insulin-degrading enzyme (abbreviated IDE) is a metalloprotease with zinc of 113 kDa that is present in bacteria, mushrooms, plants and animals. It is also described under other names such as: insulysin, insulinase, insulin glucagon protease, insulin-specific protease, FLJ35968, insulin protease. IDE is a secreted enzyme that is often associated with the plasma membrane of the cells. It is expressed in many tissues and the highest levels of IDE are found in the brain, kidneys, liver and testicles. Functional human IDE can be expressed in bacteria (Chesneau et al., 2000. Prot express & purification, 19, 91-98). IDE has been described as a biomarker of the scalps' condition (see FR2968560) or linked to healing of skin wound (Hardman et al. 2008. Gen Biol, 9:R80).

As such, according to a first of its aspects, the disclosure relates to a diagnostic method, preferably *in vitro,* of a skin presenting signs of aging, in a subject, said method comprising the following step:
a) measuring the level of expression of the gene encoding the IDE, in a skin sample of said subject.

Optionally this diagnostic method further comprises the following step:
b) based on the level of expression of the gene encoding the IDE measured in the step a), determining if the skin of said subject has signs of aging.

This disclosure also relates to a method of cosmetic treatment of a skin having signs of aging in a subject, said method comprising the following steps:
a) measuring the level of expression of the gene encoding the IDE, in a skin sample of said subject;
b) deducing from the step a) if the skin of said subject has signs of aging;
c) if the skin is identified as having signs of aging in the step b), treating said skin with a cosmetic composition allowing for the reduction and/or the slowing down of the progression of the signs of aging of the skin.

The invention is as defined by the claims.

The term "signs of aging of the skin" means here that all of the modifications of the skin that take place with age and which are sometimes not visible in the early stages. Signs of aging include marked microrelief of the skin, fine lines, appearance of wrinkles, loss of tonicity, loss of density loss of firmness, loss of elasticity, decrease in the thickness of the dermis and/or epidermis, dryness, withered skin, loss of the radiance of the skin complexion, appearance of a wizened appearance of the skin, sagging of the skin and withering of the skin.

The signs of skin aging that are detected and/or treated by the methods of the invention are marked microrelief of the skin, fine lines, and appearance of wrinkles and loss of elasticity. More preferably, the signs of skin aging that are detected and/or treated by the methods of the invention are elastosis, the appearance of crow's feet wrinkles and the appearance of wrinkles under the eyes. In particular, the appearance of dandruff is not directly related to aging and is not considered as a sign of aging.

IDE is a protein that has two isoforms of which the amino acid sequences are known and in particular correspond to the sequence with the NCBI accession code: NP_004960.2 (version from April 13, 2016) and NP_001159418.1 (version from April 13, 2016). The mRNA sequence encoding IDE is also known to those skilled in the art and particularly accessible with the NCBI accession code: M21188.1 (version from June 23, 2010).

The term "level of expression of the gene encoding IDE" denotes the level of expression of messenger RNA (mRNA) of the gene encoding IDE and/or the level of expression of IDE protein.

Preferably, the level of expression of IDE protein is measured using a specific ligand of IDE protein, such as for example an antibody, preferably monoclonal, an Fab fragment, an scFv or a nanobody, specific for this protein. The level of expression may then be measured by means of any method known to those skilled in the art, such as for example by means of an ELISA test. In particular, the measurement of the level of expression of IDE protein may be made using a miniaturized system such as a microfluidic chip, such as that described in the international application WO2011/126249, this chip containing specific ligands for IDE protein. The chip may subsequently be analyzed by means of a suitable reader in order to determine whether IDE proteins have bonded with said ligand and to reach a conclusion regarding the presence and the quantity of IDE in the skin sample.

Preferably, the level of expression of mRNA of the gene encoding IDE is measured using a complementary nucleotide sequence of the mRNA of the gene encoding IDE and specifically hybridizing with the mRNA of the gene encoding IDE or, a fragment thereof hybridizing specifically with the mRNA of the gene encoding IDE, this sequence or this fragment comprising 5 to 50 nucleotides, preferentially 10 to 20 nucleotides, or using a pair of primers or a probe of 10 to 60 nucleotides, preferentially 15 to 30 nucleotides comprising said sequence or said fragment. The level of expression may then be measured by any means known to those skilled in the art, for example by means of quantitative PCR.

Within the scope of the invention, the terms "hybridize" or "hybridization", as well-known to those skilled in the art, refer to the bonding of a nucleic acid sequence with a particular nucleotide sequence under suitable conditions, particularly under stringent conditions.

The term "stringent conditions", as used herein, corresponds to conditions which are suitable for producing bond pairs between the nucleic acids having a defined level of complementarity, while being unsuitable for the formation of pairs between the bonding nucleic acids having a lower complementarity than said defined level. The stringent conditions are dependent on hybridization and washing conditions. These conditions may be modified according to methods known to those skilled in the art. Generally, high-stringency conditions are a hybridization temperature approximately 5°C less than the melting point (Tm), preferably close to the Tm of the perfectly base-paired strands. The hybridization procedures are well-known in the art.

High-stringency conditions generally involve hybridization at a temperature of approximately 50°C to approximately 68°C in a 5x SSC/5x Denhardt's solution/1.0% SDS solution, and washing in a 0.2x SSC/0.1% SDS solution at a temperature between approximately 60°C and approximately 68°C.

According to one preferred embodiment, the skin sample of the subject used in the diagnostic and cosmetic treatment methods according to the invention, is a sample taken, preferably non-invasively, on the subject's skin, preferentially skin of the face and/or skin of the body, preferentially on the subject's face, and in particular on the subject's cheek. According to one preferred embodiment, the skin sample of the subject used in the diagnostic and cosmetic treatment methods according to the invention is not taken from the scalp. Preferably, the skin sample is obtained from the stratum corneum. Preferably, the skin sample is obtained from the skin surface. Preferably the skin sample is a sample taken on the subject's skin in an area without lesions.

The stratum corneum is the outermost layer of the epidermis, and comprises the skin surface. It is essentially made up of dead cells.

According to one embodiment, the methods according to the invention comprise the use of skin samples taken from the subject.

This step is performed non-invasively, and in particular does not require local anesthetic. For example, the sampling method may be a technique of stripping type, consisting in applying a portion of adhesive tape to the epidermis under consideration. When this adhesive tape is detached, a fraction of the skin surface is removed. These strippings are adhesive surfaces applied to the surface of the epidermis, such as Blenderm^{®} from 3M, D'squam (commercial adhesive from CuDERM), cyanoacrylate glue or the varnish "stripping" method. By virtue of these strippings, the adherent corneocytes and the content of their intercellular spaces can be sampled and subsequently subjected to extraction in order to obtain the protein content.

The taking of a sample suitable for the methods of the invention can also be carried out more directly by "washing" the skin surface, by means, for example, of accessories of the vane turbine type or of the spiral cell type as described in the patent FR 2 667 778 combined with a fluid circuit, or simply by addition/sampling of a drop of buffer at the surface of the skin. Other sampling methods suitable for implementing the invention may be methods by rubbing the upper part of the stratum corneum by means of a twin blade system or a swab.

According to one preferred embodiment, the step for taking the sample is performed by rubbing the skin surface or using an adhesive surface such as a D-squame^{®} disc. The term "subject" denotes a human being, preferably aged from 20 to 90 years, preferentially from 30 to 80 years, from 36 to 75 years and even more preferentially from 60 to 80 years. Preferably, the subject is female. Preferentially, the subject does not suffer from dermatologic lesion and/or from dermatologic disease, more preferentially the subject does not suffer from any disease.

In one particular embodiment, step (b) is carried out after comparing the level of expression of the gene encoding IDE obtained with a reference value. In one embodiment, the reference value can be determined by the mean value of the level of expression of the gene encoding IDE in a defined population, for example a population in a defined age-group and/or having a defined skin type (Caucasian, Asian, etc. or a skin with a greasy tendency, dry skin or having a particular phototype defined according to the Fitzpatrick classification). Preferably the reference value is determined by the mean value of the level of expression of the gene encoding IDE in a Caucasian, and/or in a population having a phototype II or III according to the Fitzpatrick classification.

In one particular embodiment, the reference value is determined by the mean value of the level of expression of the gene encoding IDE in women aged from 30 to 80 years, from 36 to 75 years and even more preferably from 60 to 80 years. In one particular embodiment, the reference value is between 9.0 and 13.0 ng/ml of IDE protein and preferably the reference value is between 9.35 and 12.65 ng/ml of IDE protein. In another embodiment, the reference value is the optimal threshold value determined by ROC analysis. According to the invention, a reference value may be determined by a plurality of samples, preferably more than 50, 100, 200, 300 or 500 samples.

The term "comparison" refers to the fact of determining whether the level of expression of the gene encoding IDE is essentially identical to or is different from a reference value. Preferably, the level of expression of the gene encoding IDE is considered to be different from a reference value if the observed difference is statistically significant. If the difference is not statistically significant, the level of expression of the gene encoding IDE and the reference value are essentially identical.

On the basis of this comparison, it is possible to determine whether the skin displays signs of aging. Typically, the skin is considered to display signs of aging when the level of expression of the gene encoding IDE is significantly lower than the reference value and the skin is not considered to display signs of aging when the level of expression of the gene encoding IDE is essentially identical or significantly greater than the reference value.

The term "cosmetic composition suitable for reducing and/or slowing the progression of the signs of aging of the skin" denotes any cosmetic composition known to those skilled in the art suitable for reducing and/or slowing the progression of the signs of aging of the skin such as marked microrelief of the skin, fine lines, appearance of wrinkles and loss of elasticity, and in particular such as elastose, the appearance of crow's feet wrinkles and the appearance of wrinkles under the eyes.

The disclosure also relates to a method for identifying a compound suitable for reducing and/or slowing the progression of the signs of aging of a skin, said method comprising the following steps:
a) measuring the level of expression of the gene encoding the IDE, in a skin sample exposed to said candidate compound;
b) comparing said level of expression to the level of expression in a sample of said skin not exposed to said compound;
c) identifying said candidate compound as a compound suitable for reducing and/or slowing the progression of the signs of aging of a skin when an increase in the level of expression of the gene encoding IDE in the skin sample exposed to said candidate compound is detected, with respect to the level of expression of the gene encoding IDE in the skin sample not exposed to the candidate compound.

According to one preferred embodiment, the skin sample used in the method for identifying a compound suitable for reducing and/or slowing the progression of the signs of aging of the skin according to the invention is a skin cell culture, an epidermis culture, a reconstructed whole skin, a human skin explant, or a skin sample from a subject as defined above.

Preferentially, the methods according to the invention comprise a step for treating the sample so as to prepare the measurement of the level of expression of the gene encoding IDE. For example, the treated sample is a soluble protein extract. In particular, if the skin sample has been obtained by a noninvasive method in particular taken by tape-stripping of the stratum corneum using an adhesive surface such as a D-squame^{®} disc, the soluble protein extract may be obtained using a protein extraction system such as that described in the international application WO2015/009085 which is suitable for bringing the D-squame samples into contact with a minimal volume of extraction buffer and thereby obtaining a soluble protein extract.

The present disclosure, but not part of the claimed invention, also relates to a kit comprising:
a) a means for measuring the level of expression of the gene encoding the IDE in a skin sample, and
b) an instruction leaflet.

The term "means for measuring the expression of the gene encoding IDE" denotes a specific ligand of IDE protein, such as for example an antibody, preferably monoclonal, an Fab fragment, an scFv or a nanobody, specific for this protein or a microfluidic chip such as that described in the international application WO2011/126249, this chip containing specific ligands for IDE protein. Alternatively is meant a complementary nucleotide sequence of the mRNA of the gene encoding IDE and specifically hybridizing with the mRNA of the gene encoding IDE or, a fragment thereof hybridizing specifically with the mRNA of the gene encoding IDE, this sequence or this fragment comprising 5 to 50 nucleotides, preferentially 10 to 20 nucleotides, or a pair of primers or a probe of 10 to 60 nucleotides, preferentially 15 to 30 nucleotides comprising said sequence or said fragment.

According to one embodiment, the kit described herein also comprises a standard range for the expression of the gene encoding IDE.

According to one embodiment, the kit described herein also comprises means for taking the skin sample such as an adhesive surface such as a D-squame^{®} disc or Blenderm^{®} from 3M, cyanoacrylate glue or the varnish described before, accessories of the vane turbine type or of the spiral cell type as described in the patent FR 2 667 778, a twin blade system or a swab. Preferably means for taking the skin sample is an adhesive surface such as a D-squame^{®} disc or Blenderm^{®} from 3M, cyanoacrylate glue or a varnish as described before.

The present invention further relates to the ex vivo use of a kit for identifying a compound suitable for reducing and/or slowing the progression of the signs of aging of a skin.

The present invention will be illustrated by the following example.

### Example:

Wrinkles of the crow's feet, wrinkles under the eyes and elastosis were evaluated on the eyes and the entire face of 376 women aged 36 to 75 years. The level of protein expression of the gene encoding IDE was also evaluated on these women's cheeks, by means of a D-squame sample wherein the soluble protein extract was analyzed using an ELISA test conducted on a chip.

After having discretised the signs of aging, the statistical analysis of the results obtained shows a correlation between the presence of these signs of aging and the level of expression of IDE.

**Table 1:**

| **clinical signs of aging** | **P values** | **Threshol d (ng/ml)** | **AUC*** | **Sensitivity** | **Specificity** |
|---|---|---|---|---|---|
| Wrinkles of the crow's feet | <0.001 | 11.0 | 64.36% | 66.49% | 57.74% |
| Wrinkles under the eyes | <0.001 | 11.0 | 61.25% | 63.08% | 54.66% |
| Elastosis | <0.001 | 11.0 | 60.21% | 63.95% | 54.89% |

| | | | | | |
|---|---|---|---|---|---|
| **AUC: area under the curve* | | | | | |

The statistical analysis demonstrates an association between the concentrations in IDE and the following signs of aging: 1- wrinkles of the crow's feet, 2- wrinkles under the eye and 3- elastosis, with p values <0.001 (coming from logistic regression models). A decrease in the level of expression of the gene encoding IDE is therefore characteristic of the appearance of signs of aging and in particular of elastosis, the appearance of wrinkles of the crow's feet and the appearance of wrinkles under the eyes. The analysis of the results moreover made it possible to define thresholds between 9.35 and 12.65 ng/ml which could therefore be used as reference values.

Several statistical methods were used to evaluate the performance of the univariate models of predictions of these clinical signs with the IDE concentrations, in particular by the determining of the AUC.

## Claims

1. Diagnostic method of a skin having signs of aging, in a subject, said method comprising the following steps:
a) measuring the level of expression of the gene encoding the insulin-degrading enzyme (IDE), in a skin sample of said subject;
wherein the signs of skin aging are marked microrelief of the skin, fine lines, appearance of wrinkles and loss of elasticity.

2. Method of cosmetic treatment of a skin having signs of aging in a subject, said method comprising the following steps:
a) measuring the level of expression of the gene encoding the IDE, in a skin sample of said subject;
b) deducing from the step a) if the skin of said subject has signs of aging;
c) if the skin is identified as having signs of aging in the step b), treating said skin with a cosmetic composition allowing for the reduction and/or the slowing down of the progression of the signs of aging of the skin;
wherein the signs of skin aging are marked microrelief of the skin, fine lines, appearance of wrinkles and loss of elasticity.

3. Method of identifying a compound suitable for reducing and/or slowing the progression of the signs of aging of a skin, said method comprising the following steps:
a) measuring the level of expression of the gene encoding the IDE, in a skin sample exposed to said candidate compound;
b) comparing said level of expression measured in step a) to the level of expression of the gene encoding IDE in a skin sample not exposed to said compound;
c) identifying said candidate compound as a compound suitable for reducing and/or slowing the progression of the signs of aging of a skin when an increase in the level of expression of the gene encoding IDE in the skin sample exposed to said candidate compound is detected, with respect to the level of expression of the gene encoding IDE in the skin sample not exposed to the candidate compound;
wherein the signs of skin aging are marked microrelief of the skin, fine lines, appearance of wrinkles and loss of elasticity.

4. Method according to claim 3, wherein said skin sample is a skin cell culture, an epidermis culture, a reconstructed whole skin, a human skin explant, or a skin sample from a subject.

5. Method according to one of claims 1 to 4, wherein the skin sample has been taken by rubbing the skin surface or using an adhesive surface.

6. Method according to one of claims 1 to 5, wherein the level of expression of the gene encoding IDE is determined by the level of expression of mRNA of said gene or by measuring the level of expression of the protein encoded by said gene.

7. Method according to claim 6, wherein the level of expression of the protein encoded by the gene encoding IDE is measured using at least one specific ligand of IDE protein.

8. Method according to one of claims 1 to 7, wherein the signs of skin aging that are diagnosed and/or treated by the methods are elastosis, the appearance of crow's feet wrinkles and the appearance of wrinkles under the eyes.

9. Ex vivo use of a kit for detecting signs of aging of a skin, said kit comprising:
a) a means for measuring, in a skin sample, the level of expression of the gene encoding IDE,
b) an instruction leaflet,
c) a means for taking the skin sample and
d) a standard range for the expression of the gene encoding IDE;
wherein the signs of skin aging that are detected are marked microrelief of the skin, fine lines, appearance of wrinkles and loss of elasticity.

10. Use according to claim 9, wherein the means for measuring, in a skin sample, of the level of expression of the gene encoding IDE is a specific ligand of IDE or a complementary nucleotide sequence of the mRNA or a fragment of the latter, a pair of primers or a specific probe of the mRNA of the IDE.

11. Use of a kit for identifying a compound suitable for reducing and/or slowing the progression of the signs of aging of a skin, said kit comprising:
a) a means for measuring, in a skin sample, the level of expression of the gene encoding IDE,
b) an instruction leaflet,
c) a means for taking the skin sample and
d) a standard range for the expression of the gene encoding IDE;
wherein the signs of skin aging are marked microrelief of the skin, fine lines, appearance of wrinkles and loss of elasticity.

12. Use according to claim 11, wherein the means for measuring, in a skin sample, of the level of expression of the gene encoding IDE is a specific ligand of IDE or a complementary nucleotide sequence of the mRNA or a fragment of the latter, a pair of primers or a specific probe of the mRNA of the IDE.

## Patentansprüche

1. Verfahren zur Diagnose einer Haut, die Anzeichen von Alterung aufweist, bei einem Subjekt, das Verfahren umfassend die folgenden Schritte:
a) Messen des Expressionsgrads des Gens, das für das insulinabbauende Enzym (IDE) kodiert, in einer Hautprobe des Subjekts;
wobei die Anzeichen von Hautalterung ein ausgeprägtes Mikrorelief der Haut, feine Linien, ein Auftreten von Falten und Elastizitätsverlust sind.

2. Verfahren zur kosmetischen Behandlung einer Haut, die Anzeichen von Alterung aufweist, das Verfahren umfassend die folgenden Schritte:
a) Messen des Expressionsgrads des Gens, das für das IDE kodiert, in einer Hautprobe des Subjekts;
b) Ableiten aus dem Schritt a), ob die Haut des Subjekts Anzeichen von Alterung aufweist;
c) wenn die Haut in Schritt b) so identifiziert wird, dass sie Anzeichen von Alterung aufweist, Behandeln der Haut mit einer kosmetischen Zusammensetzung, die es ermöglicht, das Fortschreiten der Zeichen der Hautalterung zu verringern und/oder zu verlangsamen;
wobei die Anzeichen von Hautalterung ein ausgeprägtes Mikrorelief der Haut, feine Linien, ein Auftreten von Falten und Elastizitätsverlust sind.

3. Verfahren zum Identifizieren einer Verbindung, die zur Verringerung und/oder Verlangsamung des Fortschreitens der Zeichen der Anzeichen von Alterung der Haut geeignet ist, das Verfahren umfassend die folgenden Schritte:
a) Messen des Expressionsgrads des Gens, das für IDE kodiert, in einer Hautprobe, die der Kandidatenverbindung ausgesetzt war;
b) Vergleichen des in Schritt a) gemessenen Expressionsgrads mit dem Expressionsgrad des Gens, das für IDE kodiert, in einer Hautprobe, die der Verbindung nicht ausgesetzt war;
c) Identifizieren der Kandidatenverbindung als eine Verbindung, die zur Verringerung und/oder Verlangsamung der Verschlechterung der Anzeichen von Alterung der Haut geeignet ist, wenn ein Anstieg des Expressionsgrads des Gens, das für IDE kodiert, in der Hautprobe, die der Kandidatenverbindung ausgesetzt ist, in Bezug auf den Expressionsgrad des Gens, das für IDE kodiert, in der Hautprobe, die der Kandidatenverbindung nicht ausgesetzt ist, erfasst wird;
wobei die Anzeichen von Hautalterung ein ausgeprägtes Mikrorelief der Haut, feine Linien, ein Auftreten von Falten und Elastizitätsverlust sind.

4. Verfahren nach Anspruch 3, wobei die Hautprobe eine Hautzellkultur, eine Epidermiskultur, eine rekonstruierte ganze Haut, ein menschliches Hautexplantat oder eine Hautprobe von einem Subjekt ist.

5. Verfahren nach einem der Ansprüche 1 bis 4, wobei die Hautprobe entnommen wurde durch
Abreiben der Hautoberfläche oder unter Verwendung einer Klebefläche.

6. Verfahren nach einem der Ansprüche 1 bis 5, bei dem das Expressionsgrad des Gens, das für IDE kodiert, durch den Expressionsgrad von mRNA des Gens oder durch Messen des Expressionsgrads des durch das Gen kodierten Proteins bestimmt wird.

7. Verfahren nach Anspruch 6, wobei der Expressionsgrad des Proteins, das durch das Gen kodiert ist, das für IDE kodiert, unter Verwendung mindestens eines spezifischen Liganden von IDE-Protein gemessen wird.

8. Verfahren nach einem der Ansprüche 1 bis 7, wobei die Anzeichen von Hautalterung, die durch die Verfahren diagnostiziert und/oder behandelt werden, Elastose, das Auftreten von Krähenfußfalten und das Auftreten von Falten unter den Augen sind.

9. Ex-vivo-Verwendung eines Kits zum Erfassen von Anzeichen von Alterung einer Haut, das Kit umfassend:
a) eine Einrichtung zum Messen des Expressionsgrads des Gens, das für IDE kodiert, in einer Hautprobe,
b) einen Beipackzettel,
c) eine Einrichtung zum Entnehmen der Hautprobe und
d) einen Standardbereich für die Expression des Gens, das für IDE kodiert;
wobei die erfassten Anzeichen von Hautalterung ein ausgeprägtes Mikrorelief der Haut, feine Linien, das Auftreten von Falten und Elastizitätsverlust sind.

10. Verwendung nach Anspruch 9, **dadurch gekennzeichnet, dass** die Einrichtung zum Messen des Expressionsgrads des für IDE kodierenden Gens in einer Hautprobe ein spezifischer Ligand von IDE oder eine komplementäre Nukleotidsequenz der mRNA oder ein Fragment der letzteren, ein Primerpaar oder eine spezifische Sonde der mRNA des IDE ist.

11. Verwendung eines Kits zum Identifizieren einer Verbindung, die zur Verringerung und/oder Verlangsamung des Fortschreitens der Zeichen des Alterns der Haut geeignet ist, das Kit umfassend:
a) eine Einrichtung zum Messen des Expressionsgrads des Gens, das für IDE kodiert, in einer Hautprobe,
b) einen Beipackzettel,
c) eine Einrichtung zum Entnehmen der Hautprobe und
d) einen Standardbereich für die Expression des Gens, das für IDE kodiert;
wobei die Anzeichen von Hautalterung ein ausgeprägtes Mikrorelief der Haut, feine Linien, ein Auftreten von Falten und Elastizitätsverlust sind.

12. Verwendung nach Anspruch 11, **dadurch gekennzeichnet, dass** die Einrichtung zum Messen des Expressionsgrads des für IDE kodierenden Gens in einer Hautprobe ein spezifischer Ligand von IDE oder eine komplementäre Nukleotidsequenz der mRNA oder ein Fragment der letzteren, ein Primerpaar oder eine spezifische Sonde der mRNA von IDE ist.

## Revendications

1. Méthode de diagnostic d'une peau présentant des signes de vieillissement, chez un sujet, ladite méthode comprenant les étapes suivantes :
a) mesurer le niveau d'expression du gène codant pour l'enzyme de dégradation de l'insuline (IDE), dans un échantillon de peau dudit sujet ;
dans laquelle les signes du vieillissement cutané sont un microrelief marqué de la peau, des ridules, l'apparition de rides et une perte d'élasticité.

2. Méthode de traitement cosmétique d'une peau présentant des signes de vieillissement chez un sujet, ladite méthode comprenant les étapes suivantes :
a) mesurer le niveau d'expression du gène codant pour l'IDE, dans un échantillon de peau dudit sujet ;
b) déduire de l'étape a) si la peau dudit sujet présente des signes de vieillissement ;
c) si la peau est identifiée comme présentant des signes de vieillissement à l'étape b), traiter ladite peau avec une composition cosmétique permettant de réduire et/ou de ralentir la progression des signes de vieillissement de la peau ;
dans laquelle les signes du vieillissement cutané sont un microrelief marqué de la peau, des ridules, l'apparition de rides et une perte d'élasticité.

3. Méthode d'identification d'un composé approprié pour réduire et/ou ralentir la progression des signes de vieillissement de la peau, ladite méthode comprenant les étapes suivantes :
a) mesurer le niveau d'expression du gène codant pour l'IDE, dans un échantillon de peau exposé audit composé candidat ;
b) comparer le niveau d'expression mesuré à l'étape a) avec le niveau d'expression du gène codant pour l'IDE dans un échantillon de peau non exposé audit composé ;
c) identifier ledit composé candidat comme un composé apte à réduire et/ou à ralentir la dégradation des qualités esthétiques de la peau lorsqu'une variation du niveau d'expression du gène codant pour l'IDE dans l'échantillon de peau exposé audit composé candidat est détectée, par rapport au niveau d'expression du gène codant pour l'IDE dans l'échantillon de peau non exposé au composé candidat ;
dans laquelle les signes du vieillissement cutané sont un microrelief marqué de la peau, des ridules, l'apparition de rides et une perte d'élasticité.

4. Méthode selon la revendication 3, dans laquelle ledit échantillon de peau est une culture de cellules de peau, une culture d'épiderme, une peau entière reconstruite, un explant de peau humaine ou un échantillon de peau provenant d'un sujet.

5. Méthode selon l'une des revendications 1 à 4, dans laquelle l'échantillon de peau a été prélevé
en frottant la surface de la peau ou en utilisant une surface adhésive.

6. Méthode selon l'une des revendications 1 à 5, dans laquelle le niveau d'expression du gène codant pour l'IDE est déterminé par le niveau d'expression de l'ARNm dudit gène ou par la mesure du niveau d'expression de la protéine codée par ledit gène.

7. Méthode selon la revendication 6, dans laquelle le niveau d'expression de la protéine codée par le gène codant pour l'IDE est mesuré à l'aide d'au moins un ligand spécifique de la protéine IDE.

8. Méthode selon l'une des revendications 1 à 7, dans laquelle les signes de vieillissement cutané diagnostiqués et/ou traités par les méthodes sont l'élastose, l'apparition de rides de la patte d'oie et l'apparition de rides sous les yeux.

9. Utilisation ex vivo d'un kit pour détecter les signes de vieillissement d'une peau, ledit kit comprenant :
a) un moyen de mesurer, dans un échantillon de peau, le niveau d'expression du gène codant pour l'IDE,
b) une notice d'instructions,
c) des moyens de prélèvement de l'échantillon de peau et
d) une gamme standard pour l'expression du gène codant pour l'IDE ;
dans laquelle les signes de vieillissement cutané détectés sont un microrelief marqué de la peau, des ridules, l'apparition de rides et une perte d'élasticité.

10. Utilisation selon la revendication 9, dans laquelle le moyen de mesure, dans un échantillon de peau, du niveau d'expression du gène codant pour l'IDE est un ligand spécifique de l'IDE ou une séquence nucléotidique complémentaire de l'ARNm ou un fragment de ce dernier, une paire d'amorces ou une sonde spécifique de l'ARNm de l'IDE.

11. Utilisation d'un kit pour identifier un composé approprié pour réduire et/ou ralentir la progression des signes de vieillissement d'une peau, ledit kit comprenant :
a) un moyen de mesurer, dans un échantillon de peau, le niveau d'expression du gène codant pour l'IDE,
b) une notice d'instructions,
c) des moyens de prélèvement de l'échantillon de peau et
d) une gamme standard pour l'expression du gène codant pour l'IDE ;
dans laquelle les signes du vieillissement cutané sont un microrelief marqué de la peau, des ridules, l'apparition de rides et une perte d'élasticité.

12. Utilisation selon la revendication 11, dans laquelle le moyen de mesure, dans un échantillon de peau, du niveau d'expression du gène codant pour l'IDE est un ligand spécifique de l'IDE ou une séquence nucléotidique complémentaire de l'ARNm ou un fragment de ce dernier, une paire d'amorces ou une sonde spécifique de l'ARNm de l'IDE.
